# EUROPEAN PATENT APPLICATION

(11) **EP 4 570 267 A1**
(43) Date of publication of application: **18.06.2025**
(21) Application number: 23862708.7
(22) Date of filing: 01.05.2023
(51) Int. Cl.: A61K 47/36

(54) **MATERIAL FOR GEL FORMATION AND GEL COMPOSITION**

(30) Priority: 07.09.2022 JP 2022141930
(71) Applicant: Kewpie Corporation, Tokyo 150-0002 (JP)
(72) Inventor: FUJIKAWA Shunichi, Chofu-shi, Tokyo 182-0002 (JP)
(74) Representative: Winter, Brandl - Partnerschaft mbB
(86) International application number: PCT/JP2023/017055
(87) International publication number: WO 2024/053159

(57) **Abstract**

One aspect of the present invention relates to a material for gel formation containing at least one component A selected from the group consisting of a modified hyaluronic acid having a thiol group and a salt thereof, and at least one component B selected from the group consisting of a modified hyaluronic acid having a maleimide group and a salt thereof.

## Description

### Technical Field

The present invention relates to a material for gel formation and a gel composition.

### Background Art

An indwelling in situ gelling material can be used as a medical material that can be administered by injection into the body, and thus has attracted attention in the fields of drug carriers and tissue engineering as it may enable a minimally invasive treatment. In addition to gelling of an in situ gelling material in a relatively short time in response to a change in temperature, pH, or a chemical substance, biocompatibility and safety are also important issues.

Among various gel-forming materials, hyaluronic acid is excellent in biocompatibility and safety to the human body. As a composition for obtaining a hydrogel using hyaluronic acid, for example, Patent Literature 1 discloses a composition containing one or more hydrogel-forming polymers having an intrinsic viscosity [η] of at least 3 dL/g in the composition as measured with an Ubbelohde viscometer, in which the concentration C_{T} of the hydrogel-forming polymer in the composition is at most about 5 mg/ml.

However, a hydrogel obtained using the composition disclosed in Patent Literature 1 requires 10 hours or more for gelling, and is not suitable as a gel-forming material that gels in situ.

### Citation List

### Patent Literature

Patent Literature 1: Japanese Unexamined Patent Publication No. 2020-534427

### Summary of Invention

### Technical Problem

The present invention provides a gel-forming material and a gel composition that can gel in a short time using hyaluronic acid. Such a gel-forming material and a gel composition are suitable for a medical material that needs to be administered into a living body by injection and gelled in situ.

An object of one aspect of the present invention is to provide a material for gel formation capable of forming a gel in a short time, and a gel composition obtained using the material for gel formation. An object of one aspect of the present invention is to provide a novel material for in situ gel formation.

### Solution to Problem

The present inventors have found that a material for gel formation containing at least one component A selected from the group consisting of a modified hyaluronic acid having a thiol group and a salt thereof, and at least one component B selected from the group consisting of a modified hyaluronic acid having a maleimide group and a salt thereof can solve the above problems, and further can provide a gel composition containing hyaluronic acid in a short time within several tens of minutes (for example, within 30 minutes), and thus completed the present invention.

That is, the present invention relates to, for example, the following inventions.
[1] A material for gel formation, containing at least one component A selected from the group consisting of a modified hyaluronic acid having a thiol group and a salt thereof, and at least one component B selected from the group consisting of a modified hyaluronic acid having a maleimide group and a salt thereof.
[2] The material for gel formation according to [1], in which a thiol group modification rate of the component A is 5% or more and 50% or less.
[3] The material for gel formation according to [1] or [2], in which the component A has an average molecular weight of 10,000 or more and 600,000 or less.
[4] The material for gel formation according to any one of [1] to [3], in which a maleimide group modification rate of the component B is 1% or more and 30% or less.
[5] A material for in situ gel formation, containing at least one selected from the group consisting of a modified hyaluronic acid having a thiol group and a salt thereof.
[6] A material for in situ gel formation, containing at least one selected from the group consisting of a modified hyaluronic acid having a maleimide group and a salt thereof.
[7] A gel composition, containing at least one component A selected from the group consisting of a modified hyaluronic acid having a thiol group and a salt thereof, and at least one component B selected from the group consisting of a modified hyaluronic acid having a maleimide group and a salt thereof, in which the thiol group and the maleimide group are bonded to each other.

### Advantageous Effects of Invention

According to the present invention, it is possible to provide a material for gel formation capable of forming a gel in a short time and a gel composition obtained using the material for gel formation. According to the present invention, it is also possible to provide a novel material for in situ gel formation.

### Description of Embodiments

Hereinafter, modes for carrying out the present invention will be described in detail. However, the present invention is not limited to the following embodiments.

### <Features of the Present Invention>

### (Material for Gel Formation)

The present invention is characterized by providing a material for gel formation containing at least one component A selected from the group consisting of a modified hyaluronic acid having a thiol group and a salt thereof, and at least one component B selected from the group consisting of a modified hyaluronic acid having a maleimide group and a salt thereof. The gel-forming material of the present invention can be gelled in a short time within 1 hour, and can be suitably used for a medical material that needs to be gelled in situ.

### (Gel Composition)

The present invention is characterized by providing a gel composition containing at least one component A selected from the group consisting of a modified hyaluronic acid having a thiol group and a salt thereof, and at least one component B selected from the group consisting of a modified hyaluronic acid having a maleimide group and a salt thereof, in which the thiol group and the maleimide group are bonded to each other.

### <Material for Gel Formation>

The "material for gel formation" in the present description is a material containing one type or two or more types of components capable of forming a gel composition by a reaction with another component, and in the present invention, refers to a material for forming a gel composition within one hour from the start of a reaction with another component. The gel-forming material of the present invention contains at least one component A selected from the group consisting of a modified hyaluronic acid having a thiol group and a salt thereof, and at least one component B selected from the group consisting of a modified hyaluronic acid having a maleimide group and a salt thereof.

### <In Situ Gel Formation>

Since the material for gel formation of the present invention can form a gel in a short time within 1 hour, it is suitably used as a material for in situ gel formation. The "material for in situ gel formation" is a gel-forming material capable of forming a gel at a target place.

The material for gel formation of the present invention can be sterilized in a state before gel formation (for example, in a solution state). Therefore, sterilization in a state after gelling is not necessarily required, and it is possible to form a gel by administering the sterilized material for gel formation to a target place of a living body.

### <Modified Hyaluronic Acid Having Thiol Group and Salt Thereof>

The component A is a compound in which a thiol group (-SH) is introduced into at least a part of hyaluronic acid or a salt thereof. The material for gel formation may contain one type or two or more types of components A.

### (Component A Structure 1)

The component A may be a compound, in which functional groups contained in some disaccharide units of repeating units of disaccharide units constituting hyaluronic acid are at least partially substituted with a functional group containing a thiol group, or a salt thereof. The component A may be a compound, in which at least one group selected from the group consisting of a hydroxy group at the C4 position and a hydroxy group at the C6 position of N-acetylglucosamine constituting hyaluronic acid, and a hydroxy group at the C2 position, a hydroxy group at the C3 position, and -OH in a carboxy group at the C6 position of glucuronic acid constituting hyaluronic acid is substituted with a functional group containing a thiol group, or a salt thereof.

### (Component A Structure 2)

The component A may be, for example, a compound represented by the following formula (1) or a salt thereof.

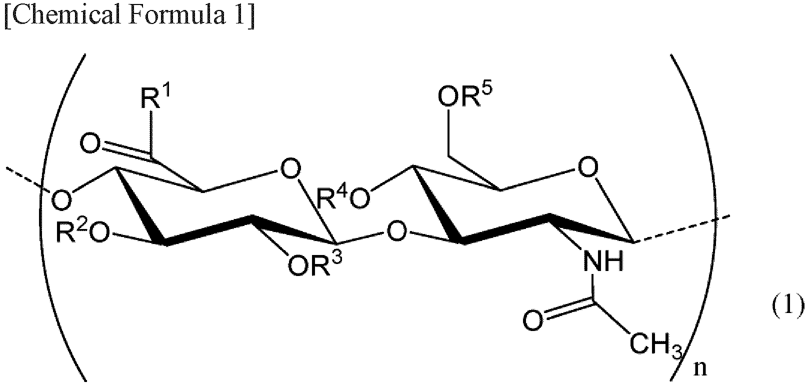

In formula (1), R¹ represents a functional group containing -OH or a thiol group, and R² to R⁵ independently represent a hydrogen atom or a functional group containing a thiol group. n represents a number of 1 or more and 7,500 or less.

### (Number and Substitution Position of Functional Groups Containing Thiol Group In Formula (1))

In formula (1), at least one of R¹ to R⁵ is a functional group containing a thiol group. In formula (1), 1 to 4, 1 to 3, or 1 to 2 of R¹ to R⁵ may be a functional group containing a thiol group, and any one of R¹ to R⁵ may be a functional group containing a thiol group. In formula (1), R¹ may be a functional group containing a thiol group, and all of R² to R⁵ may be a hydrogen atom.

### (Component A Structure 3)

The component A may be, for example, a compound represented by the following formula (1a) or a salt thereof.

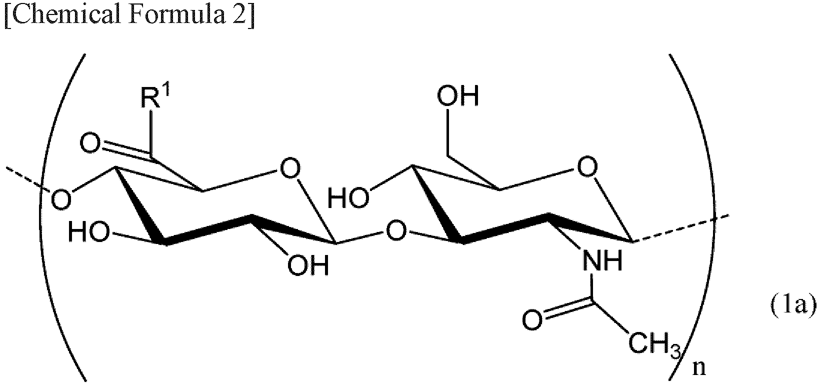

In formula (1a), R¹ and n have the same meanings as R¹ and n in formula (1), respectively.

### (Structure of Functional Group Containing Thiol Group)

The functional group containing a thiol group may be a group represented by formula (I): -X¹-Z¹-SH.

### (Structure of Z¹ in Formula (I))

Z¹ represents a divalent group, and is a group linking X¹ to SH. Z¹ may be, for example, a divalent hydrocarbon group. The divalent hydrocarbon group may be linear or branched. The number of carbon atoms in the divalent hydrocarbon group may be, for example, 1 or more, or 2 or more, and may be 10 or less, 8 or less, 6 or less, 4 or less, or 3 or less. The divalent hydrocarbon group may be, for example, an alkylene group. Z¹ may be an alkylene group having 2 or more carbon atoms. When Z¹ is an alkylene group having 2 or more carbon atoms, the elasticity of the resulting gel composition becomes more excellent. When Z¹ is an alkylene group having 3 or less carbon atoms, the solubility in water or PBS becomes more excellent.

### (Structure of X¹ in Formula (I))

X¹ may be -NH- or -O- when the bonding site is R¹. X¹ may be - (C=O)- or a single bond when the bonding site is R² to R⁵.

### (Specific Examples of Functional Group Containing Thiol Group)

The functional group containing a thiol group may be -NH-CH₂-CH₂-SH or -O-CH₂-CH₂-SH when the bonding site is R¹. The functional group containing a thiol group may be -(C=O)-CH₂-SH or -CH₂-CH₂-SH when the bonding site is R² to R⁵.

### (Salt of Modified Hyaluronic Acid Having Thiol Group)

The salt of the modified hyaluronic acid having a thiol group may be a pharmaceutically acceptable salt. Examples of the salt of the modified hyaluronic acid having a thiol group include a sodium salt, a potassium salt, and an ammonium salt.

### (Preferred Upper Limit Value of Average Molecular Weight of Component A)

The average molecular weight of the component A may be 1,500,000 or less, 1,400,000 or less, 1,300,000 or less, 1,200,000 or less, 1,100,000 or less, or 1,000,000 or less from the viewpoint of solubility in a solvent such as water or PBS. When the average molecular weight of the component A is 1,000,000 or less, the solubility in a solvent such as water or PBS becomes still more excellent. The average molecular weight of the component A is preferably 800,000 or less, 700,000 or less, 600,000 or less, 500,000 or less, 400,000 or less, 300,000 or less, or 200,000 or less from the viewpoint of solubility in a solvent such as water or PBS.

### (Preferred Lower Limit Value of Average Molecular Weight of Component A)

The average molecular weight of the component A may be 10,000 or more, 20,000 or more, 30,000 or more, 40,000 or more, 50,000 or more, 60,000 or more, 70,000 or more, or 80,000 or more from the viewpoint of obtaining a gel composition having elasticity. When the average molecular weight of the component A is 80,000 or more, the elasticity of the resulting gel composition becomes still more excellent. The average molecular weight of the component A is preferably 100,000 or more from the viewpoint of obtaining a gel composition having elasticity.

### (Preferred Numerical Range of Average Molecular Weight of Component A)

The average molecular weight of the component A may be 10,000 or more and 1,500,000 or less, 80,000 or more and 1,000,000 or less, or 100,000 or more and 800,000 or less from the viewpoint that the solubility in a solvent such as water or PBS becomes more excellent. The average molecular weight of the component A is preferably 10,000 or more and 600,000 or less, and more preferably 100,000 or more and 300,000 or less from the viewpoint that the solubility in a solvent such as water or PBS becomes more excellent.

### (Method for Measuring Average Molecular Weight)

The average molecular weight can be measured by the following method.

First, a plurality of (purified) hyaluronic acids (reference substances) having known molecular weights are subjected to a liquid chromatography analysis using a gel filtration column, and a calibration curve is prepared from the retention times thereof. Similarly, the molecular weight of the modified hyaluronic acid can be determined by subjecting the modified hyaluronic acid to be measured to a liquid chromatography analysis and determining the molecular weight using the prepared calibration curve.

### (Measurement Device and Measurement Conditions for Average Molecular Weight)

A 2690 separation module manufactured by Nihon Waters K.K. is used as a liquid chromatography analyzer. As a photodiode array, a 996 photodiode array manufactured by Nihon Waters K.K. is used. As the column, one TSK guard column PWXL (manufactured by Tosoh Corporation) and two TSK gel GMPW columns (manufactured by Tosoh Corporation) connected in series in the described order are used. In the measurement of the average molecular weight, conditions of a column temperature of 40°C, a measurement wavelength of 210 nm, a flow rate of 0.8 mL/min, a sample injection amount of 20 µL, an analysis time of 40 minutes, and a mobile phase of 0.003 mol/L phosphate buffer-0.15 mol/L NaCl (pH 7.0) are used. The details of other test conditions may be as described in the below-mentioned examples.

### (Definition of Thiol Group Modification Rate)

The thiol group modification rate of the component A means the number of thiol groups contained in one unit, where one unit is a disaccharide unit constituting hyaluronic acid, and specifically refers to the ratio (%) of the number of thiol groups contained per unit to the one unit when the one unit is taken as 100%. In the present description, the "disaccharide unit constituting hyaluronic acid" refers to one unit constituted by disaccharides (glucuronic acid and N-acetylglucosamine) that constitute hyaluronic acid and are bonded adjacent to each other.

### (Method for Measuring Thiol Group Modification Rate)

The thiol group modification rate of the component A can be measured using ¹H-NMR. Specific examples of the method for preparing a measurement sample and the measurement method are as described in the below-mentioned examples.

### (Preferred Lower Limit Value of Thiol Group Modification Rate)

The thiol group modification rate of the component A may be 1% or more, 2% or more, 3% or more, 4% or more, 5% or more, 6% or more, 7% or more, 8% or more, 9% or more, or 10% or more from the viewpoint of gel-forming ability. When the thiol group modification rate of the component A is 5% or more, the gel-forming ability becomes still more excellent even when the concentration of the modified hyaluronic acid in the gel-forming material is as low as less than 1%.

### (Preferred Upper Limit Value of Thiol Group Modification Rate)

The thiol group modification rate of the component A may be 80% or less, 75% or less, 70% or less, 65% or less, 60% or less, 55% or less, or 50% or less from the viewpoint of solubility in a solvent such as water or PBS. When the thiol group modification rate of the component A is 50% or less, the solubility in a solvent such as water or PBS becomes still more excellent.

### (Preferred Numerical Range of Thiol Group Modification Rate)

The thiol group modification rate of the component A is preferably 1% or more and 80% or less, and more preferably 5% or more and 50% or less, or 6% or more and 45% or less from the viewpoint that the gel-forming ability in a short time becomes more excellent.

### (Form of Component A)

In the material for gel formation, the component A may be in a liquid form or a solid form (for example, a powder form). The material for gel formation may contain a solution containing the component A and a solvent or a powder containing the component A. Examples of the solvent include water and a buffer. Examples of the buffer include a phosphate buffer. The solvent may be a phosphate buffer because a gel is easily obtained in a shorter time.

### (Solution Containing Component A)

In the solution containing the component A, the content of the component A may be 0.1 mg or more, 0.5 mg or more, 1 mg or more, or 1.5 mg or more, and may be 25 mg or less, 15 mg or less, 10 mg or less, 7.5 mg or less, or 5 mg or less with respect to 1 mL of the solvent from the viewpoint of gel-forming ability.

### (Method for Producing Component A)

The component A can be obtained, for example, by a method including a reaction step A of allowing hyaluronic acid to react with a raw material compound A containing a group capable of reacting with a functional group in hyaluronic acid and a thiol group in a reaction liquid containing hyaluronic acid and the raw material compound A. The method for producing the component A may further include a post-treatment step A of subjecting the reaction product obtained in the reaction step A to a post-treatment.

### (Raw Material Compound A)

The raw material compound A may be a compound represented by formula (Ia): X²-Z¹-SH or a salt thereof. Z¹ in formula (Ia) may be a group similar to Z¹ in formula (I). In formula (Ia), X² represents a group capable of reacting with a functional group in hyaluronic acid. Examples of the group capable of reacting with a functional group in hyaluronic acid include an amino group (-NH₂). The amount of the raw material compound A used is appropriately adjusted according to the intended thiol group modification rate or the like.

### (Specific Examples of Raw Material Compound A)

Examples of the raw material compound A include cysteamine (NH₂-CH₂-CH₂-SH), 2-hydroxyethanethiol (HO-CH₂-CH₂-SH), thioglycolic acid (HOOC-CH₂-SH), 2-chloroethanethiol (Cl-CH₂-CH₂-SH), and disulfide compounds thereof.

### (Solvent in Reaction Step A)

The reaction liquid may contain a solvent. Examples of the solvent include water and a mixed solution of water and a water-soluble organic solvent such as ethanol.

### (Reactant in Reaction Step A)

The reaction liquid may contain a condensing agent. Examples of the condensing agent include a carbodiimide-based condensing agent. Examples of the carbodiimide-based condensing agent include 1-(3-dimethylaminopropyl)-3-ethylcarbodiimide hydrochloride (EDC·HCl). The reaction liquid may further contain a condensation aid in addition to the condensing agent. Examples of the condensation aid include 1-hydroxybenzotriazole (HOBT).

### (Post-treatment Step A)

As the post-treatment step A, for example, a step of precipitating the component A or a precursor thereof, a step of washing the component A or a precursor thereof, a step of allowing a precursor of the component A to react with a reducing agent, or the like may be performed. Examples of the method for precipitating the component A or a precursor thereof include a method in which an organic solvent such as ethanol is mixed with a reaction liquid obtained after the reaction step A. Examples of the method for washing the component A or a precursor thereof include a method for washing a precipitate containing the component A or a precursor thereof with an aqueous ethanol solution or the like. Examples of the reducing agent when a precursor of the component A is allowed to react with the reducing agent include tris(2-carboxyethyl)phosphine (TCEP), dithiothreitol, and mercaptoethanol.

### <Modified Hyaluronic Acid Having Maleimide Group and Salt Thereof>

The component B is hyaluronic acid, in which a maleimide group represented by the following formula is introduced into at least a part of hyaluronic acid, or a salt thereof. The material for gel formation may contain one type or two or more types of components B.

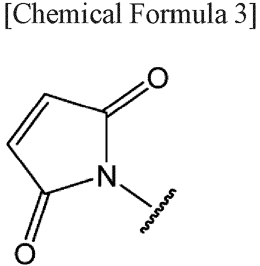

### (Component B Structure 1)

The component B may be a compound, in which functional groups contained in some disaccharide units of repeating units of disaccharide units constituting hyaluronic acid are at least partially substituted with a functional group containing a maleimide group, or a salt thereof. The component B may be a compound, in which at least one group selected from the group consisting of a hydroxy group at the C4 position and a hydroxy group at the C6 position of N-acetylglucosamine constituting hyaluronic acid, and a hydroxy group at the C2 position, a hydroxy group at the C3 position, and -OH in a carboxy group at the C6 position of glucuronic acid constituting hyaluronic acid is substituted with a functional group containing a maleimide group, or a salt thereof.

### (Component B Structure 2)

The component B may be, for example, a compound represented by the following formula (2) or a salt thereof.

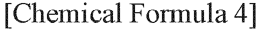

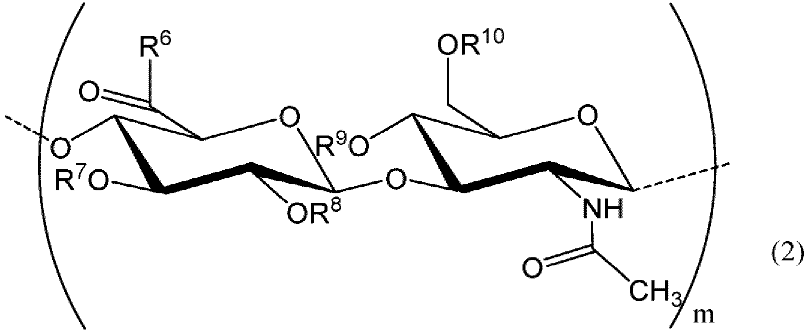

In formula (2), R⁶ represents a functional group containing -OH or a maleimide group, and R⁷ to R¹⁰ independently represent a hydrogen atom or a functional group containing a maleimide group. m represents a number of 1 or more and 7,500 or less.

### (Number and Substitution Position of Functional Groups Containing Maleimide Group)

In formula (2), at least one of R⁶ to R¹⁰ is a functional group containing a maleimide group. In formula (2), 1 to 4, 1 to 3, or 1 to 2 of R⁶ to R¹⁰ may be a functional group containing a maleimide group, and any one of R⁶ to R¹⁰ may be a functional group containing a maleimide group. In formula (2), R⁶ is a functional group containing a maleimide group, and all of R⁷ to R¹⁰ may be a hydrogen atom.

### (Component B Structure 3)

The component B may be, for example, a compound represented by the following formula (2a) or a salt thereof.

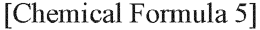

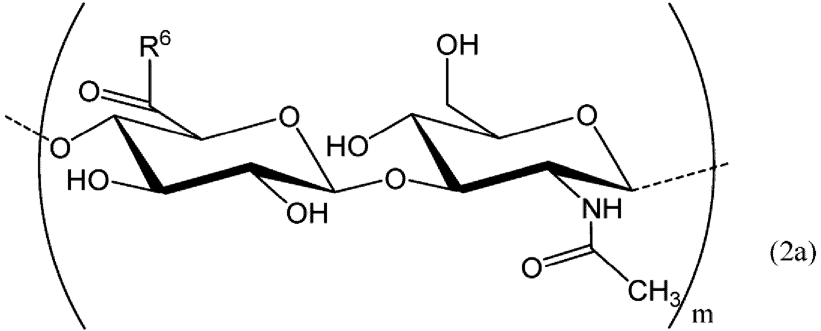

In formula (2a), R⁶ and m have the same meanings as R⁶ and m in formula (2), respectively.

### (Structure of Functional Group Containing Maleimide Group)

The functional group containing a maleimide group may be a group represented by formula (II): -X²-Z²-Y. Y represents a maleimide group.

### (Structure of Z² in Formula (II))

Z² represents a divalent group, and is a group linking X² to Y. Z² may be, for example, a divalent hydrocarbon group. The divalent hydrocarbon group may be linear or branched. The number of carbon atoms in the divalent hydrocarbon group may be, for example, 1 or more, or 2 or more, and may be 10 or less, 8 or less, 6 or less, 4 or less, or 3 or less. The divalent hydrocarbon group may be, for example, an alkylene group. Z² may be an alkylene group having 2 or more carbon atoms. When Z² is an alkylene group having 2 or more carbon atoms, the elasticity of the resulting gel composition becomes more excellent. When Z² is an alkylene group having 3 or less carbon atoms, the solubility in a solvent such as water or PBS becomes more excellent.

### (Structure of X² in Formula (II))

X² may be -NH- or -O- when the bonding site is R⁶. X² may be - (C=O)- or a single bond when the bonding site is R⁷ to R¹⁰.

### (Specific Examples of Functional Group Containing Maleimide Group)

The functional group containing a maleimide group may be -NH-CH₂-CH₂-Y, -NH-CH₂-Y, -NH-CH₂-CH₂-CH₂-Y, or -O-CH₂-CH₂-Y when the bonding site is R⁶. The functional group containing a maleimide group may be -(C=O)-CH₂-Y or -CH₂-CH₂-Y when the bonding site is R⁷ to R¹⁰.

### (Salt of Modified Hyaluronic Acid Having Maleimide Group)

The salt of the modified hyaluronic acid having a maleimide group may be a pharmaceutically acceptable salt. Examples of the salt of the modified hyaluronic acid having a maleimide group include a sodium salt, a potassium salt, and an ammonium salt.

### (Preferred Upper Limit Value of Average Molecular Weight of Component B)

The average molecular weight of the component B may be 100,000 or less, 90,000 or less, 80,000 or less, 70,000 or less, 60,000 or less, or 50,000 or less from the viewpoint of solubility in a solvent such as water or PBS. When the average molecular weight of the component B is 50,000 or less, the solubility in a solvent such as water or PBS becomes still more excellent. The average molecular weight of the component B is preferably 10,000 or less from the viewpoint of solubility in a solvent such as water or PBS.

### (Preferred Lower Limit Value of Average Molecular Weight of Component B)

The average molecular weight of the component B may be 2,000 or more, 3,000 or more, 5,000 or more, 6,000 or more, 7,000 or more, or 8,000 or more from the viewpoint of gel-forming ability. When the average molecular weight of the component B is 8,000 or more, the gel-forming ability becomes more excellent even when the concentration of the modified hyaluronic acid in the gel-forming material is as low as less than 1%.

### (Preferred Numerical Range 1 of Average Molecular Weight of Component B)

The average molecular weight of the component B may be 2,000 or more and 100,000 or less, 8,000 or more and 50,000 or less, 9,000 or more and 30,000 or less, and is preferably 5,000 or more and 20,000 or less from the viewpoint that the solubility in a solvent such as water or PBS becomes more excellent.

### (Method for Measuring Average Molecular Weight of Component B)

The average molecular weight of the component B can be measured in the same manner as the average molecular weight of the component A.

### (Definition of Maleimide Group Modification Rate)

The maleimide group modification rate of the component B means the number of maleimide groups contained in one unit, where one unit is a disaccharide unit constituting hyaluronic acid, and specifically refers to the ratio (%) of the number of maleimide groups contained per unit to the one unit when the one unit is taken as 100%.

### (Method for Measuring Maleimide Group Modification Rate)

The maleimide group modification rate of the component B can be measured using ¹H-NMR. Specific examples of the method for preparing a measurement sample and the measurement method are as described in the below-mentioned examples.

### (Preferred Lower Limit Value of Maleimide Group Modification Rate)

The maleimide group modification rate of the component B may be 1% or more, 2% or more, 3% or more, 4% or more, 5% or more, 8% or more, 10% or more, or 12% or more from the viewpoint of gel-forming ability. When the maleimide group modification rate of the component B is 5% or more, the gel-forming ability becomes more excellent even when the concentration of the modified hyaluronic acid in the gel-forming material is as low as less than 1%. The maleimide group modification rate of the component B is preferably 15% or more from the viewpoint that the gel-forming ability in a short time becomes more excellent.

### (Preferred Upper Limit Value of Maleimide Group Modification Rate)

The maleimide group modification rate of the component B may be 80% or less, 70% or less, 60% or less, or 50% or less from the viewpoint of solubility in a solvent such as water or PBS. When the maleimide group modification rate of the component B is 50% or less, the solubility in a solvent such as water or PBS becomes still more excellent. The maleimide group modification rate of the component B is preferably 30% or less, and more preferably 20% or less from the viewpoint of solubility in a solvent such as water or PBS.

### (Preferred Numerical Range of Maleimide Group Modification Rate)

The maleimide group modification rate of the component B may be 1% or more and 80% or less, 5% or more and 50% or less, or 10% or more and 45% or less from the viewpoint that the solubility in a solvent such as water or PBS becomes more excellent. The maleimide group modification rate of the component B is preferably 1% or more and 30% or less from the viewpoint that the solubility in a solvent such as water or PBS becomes more excellent, and more preferably 15% or more and 25% or less from the viewpoint that the solubility in a solvent such as water or PBS becomes more excellent.

### (Form of Component B)

In the material for gel formation, the component B may be in a liquid form or a solid form (for example, a powder form). The material for gel formation may contain a solution containing the component B and a solvent or a powder containing the component B. Examples of the solvent include water and a buffer. Examples of the buffer include a phosphate buffer. The solvent may be a phosphate buffer because a gel is easily obtained in a shorter time.

### (Solution Containing Component B)

In the solution containing the component B, the content of the component B may be 0.1 mg or more, 1 mg or more, or 1.5 mg or more, and may be 25 mg or less, 15 mg or less, 10 mg or less, 7.5 mg or less, or 5 mg or less with respect to 1 mL of the solvent from the viewpoint of gel-forming ability.

### (Method for Producing Component B)

The component B can be obtained, for example, by a method including a reaction step B of allowing hyaluronic acid to react with a raw material compound B containing a group capable of reacting with a functional group in hyaluronic acid and a maleimide group in a reaction liquid containing hyaluronic acid and the raw material compound B. The method for producing the component B may further include a post-treatment step B of subjecting the reaction product obtained in the reaction step B to a post-treatment.

### (Raw Material Compound B)

The raw material compound B may be a compound represented by formula (IIa): X²-Z²-Y or a salt thereof. Z² in formula (IIa) may be a group similar to Z² in formula (II). In formula (IIa), X² represents a group capable of reacting with a functional group in hyaluronic acid. Examples of the group capable of reacting with a functional group in hyaluronic acid include an amino group (-NH₂). The amount of the raw material compound B used is appropriately adjusted according to the intended maleimide group modification rate or the like.

### (Specific Examples of Raw Material Compound B)

Examples of the raw material compound B include N-(2-aminoethyl)maleimide or a salt thereof (for example, a hydrochloride), N-(2-hydroxyethyl)maleimide, 2-maleimidoacetic acid, and N-(2-chloroethyl)maleimide.

### (Solvent and Reactant in Reaction Step B)

As the solvent and the reactant in the reaction step B, the solvent and the reactant exemplified for the reaction step A can be used.

### (Post-treatment Step B)

Examples of the post-treatment step B include a step of precipitating the component B, a step of washing the component B, and the like. Examples of the method for precipitating the component B include a method in which an organic solvent such as ethanol is mixed with a reaction liquid obtained after the reaction step B. Examples of the method for washing the component B include a method for washing a precipitate containing the component B with an aqueous ethanol solution or the like.

### <Medicinal substance>

The material for gel formation may further contain a medicinal substance. The medicinal substance may be a water-soluble medicinal substance. Examples of the medicinal substance include a peptide, a protein, and a gene.

### <Other Components>

The material for gel formation may contain the component A, the component B, and other components that do not correspond to the medicinal substance. Examples of other components include water, ethanol, a phosphate, and inorganic salts such as sodium chloride and potassium chloride.

### <Method for Gelling Material for Gel Formation>

A gel is formed by mixing the component A and the component B which are the material for gel formation of the present invention. Examples of the gelling method include a method of mixing a solution containing the component A and a solution containing the component B, a method of mixing a powder containing the component B with a solution containing the component A, and a method of mixing a powder containing the component A with a solution containing the component B. The gelling method is preferably a method of mixing a solution containing the component A and a solution containing the component B from the viewpoint of preventing generation of lumps during gel formation.

### (Mass Ratio of Component B to Component A)

The ratio of the mass of the component B to the mass of the component A (mass of component B/mass of component A) may be 0.01 or more, 0.05 or more, 0.06 or more, 0.07 or more, 0.08 or more, 0.09 or more, or 0.1 or more, and may be 10.0 or less, 9.0 or less, 8.0 or less, 7.0 or less, 6.0 or less, or 5.0 or less.

### (Temperature Conditions for Gelling)

The temperature (gelling temperature) at the time of gelling of the material for gel formation may be 0°C or higher, 10°C or higher, or 15°C or higher, and may be 40°C or lower, or 35°C or lower, or may be room temperature. In the present description, room temperature means 23°C.

### <Method for Administering Material for Gel Formation>

Examples of the method for administering the material for gel formation include administration by injection. When the material for gel formation is administered into a living body by injection, the administration can be performed using a syringe including a syringe body capable of separately storing a first component containing at least the component A and a second component containing at least the component B, and a syringe needle. When the syringe is used, the first component and the second component are mixed immediately before administration into a living body (in the syringe needle of the syringe), and a gel composition is formed at the site of administration.

### <Formation of Gel having Biocompatibility>

The material for gel formation forms a gel composition by a reaction between the component A and the component B, and therefore can form a gel composition having biocompatibility.

### <Material for In Situ Gel Formation 1>

The material for in situ gel formation of one embodiment contains the component A described above. The material for in situ gel formation containing the component A can form a gel in situ by the reaction with the component B described above.

### <Material for In Situ Gel Formation 2>

The material for in situ gel formation of one embodiment contains the component B described above. The material for in situ gel formation containing the component B can form a gel in situ by the reaction with the component A described above.

### <Gel Composition>

The gel composition contains the component A and the component B, and a thiol group and a maleimide group are bonded to each other. That is, the gel composition contains a partial structure represented by the following formula (II) formed by a reaction between a thiol group and a maleimide group. The gel composition is formed from the component A and the component B, and therefore has biocompatibility.

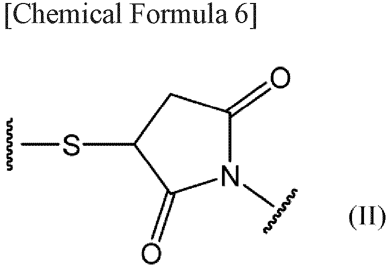

### (Mode of Gel Composition)

As a specific mode of the gel composition, the specific mode of the material for gel formation described above can be applied. The gel composition may contain the above-mentioned medicinal substance and other components. The gel composition does not exclude the inclusion of impurities inevitably contained with the preparation.

### <Method for Producing Gel Composition>

The gel composition can be obtained by a method including a step of gelling the material for gel formation described above. The gelling step can be performed, for example, by a method including mixing the above-mentioned component A and the above-mention component B. The conditions for gelling may be as described above.

### <Applications of Gel Composition>

Examples of applications of the gel composition include a locally sustained-release material of a medicinal substance, an adhesion-preventing material, a hemostatic material (for example, a hemostatic material for endoscopic surgery), a wound dressing, a scaffold material for cell culture, a skin filler, and a joint injection material.

### <Sustained-Release Preparation>

A sustained-release preparation contains a first component containing the component A, a second component containing the component B, and a medicinal substance. The medicinal substance may be contained in either one or both of the first component and the second component. Other components described above may be contained in either one or both of the first component and the second component. The sustained-release preparation contains the first component and the second component in a state before mixing. By mixing the first component and the second component immediately before or at the same time as administration to a living body, a gel composition containing a medicinal substance is formed at a target site of the living body. When the target site is in a living body, the gel composition containing a medicinal substance undergoes hydrolysis or the like in the living body, and the gel composition is disintegrated, so that the medicinal substance is gradually released.

### <Use of Component A and/or Component B for Producing Material for Gel Formation>

As one embodiment of the present invention, the use (application) of the component A and the component B for producing the material for gel formation is provided. As one embodiment of the present invention, the use of the component A for producing the material for in situ gel formation and the use of the component B for producing the material for in situ gel formation are provided. In these embodiments, the modes of the material for gel formation described above can be applied without limitation.

### <Component A and/or Component B Used for Gel Formation>

As one embodiment of the present invention, the component A and the component B used for gel formation are provided. As one embodiment of the present invention, the component A used for in situ gel formation and the component B used for in situ gel formation are provided. In these embodiments, the modes of the material for gel formation described above can be applied without limitation.

### Examples

Hereinafter, the present invention will be described more specifically with reference to examples and the like. However, the present invention is not limited to the following examples.

### [Example 1]

### <Method for Producing Modified Hyaluronic Acid Having Thiol Group>

In 50 mL of pure water, 0.5 g of hyaluronic acid (Hyabest (S) LF-P manufactured by Kewpie Corporation, average molecular weight: about 300,000) was dissolved to obtain a hyaluronic acid solution. To the hyaluronic acid solution, 106 mg of 1-hydroxybenzotriazole (HOBT), 120 mg of 1-(3-dimethylaminopropyl)-3-ethylcarbodiimide hydrochloride (EDC·HCl), and 141 mg of cystamine dihydrochloride were added, and a reaction was allowed to proceed overnight. To the resulting reaction liquid, 150 mL of ethanol was slowly added to precipitate a modified hyaluronic acid, which was washed three times with 50 mL of 80% ethanol to obtain a precursor of a modified hyaluronic acid having a thiol group. In 50 mL of pure water, 0.5 g of the obtained precursor was dissolved, and 180 mg of tris(2-carboxyethyl)phosphine (TCEP) was added thereto, and a reaction was allowed to proceed overnight. To the resulting reaction liquid, 150 mL of ethanol was slowly added to precipitate a modified hyaluronic acid, which was washed three times with 100 mL of 80% ethanol to obtain a modified hyaluronic acid having a thiol group (thiol group-containing modified HA).

### [Example 2]

### <Method for Producing Modified Hyaluronic Acid Having Thiol Group>

In 50 mL of pure water, 0.5 g of hyaluronic acid (Hyabest (S) LF-P manufactured by Kewpie Corporation, average molecular weight: about 300,000) was dissolved to obtain a hyaluronic acid solution. To the hyaluronic acid solution, 53 mg of 1-hydroxybenzotriazole (HOBT), 60 mg of 1-(3-dimethylaminopropyl)-3-ethylcarbodiimide hydrochloride (EDC·HCl), and 71 mg of cystamine dihydrochloride were added, and a reaction was allowed to proceed overnight. To the resulting reaction liquid, 150 mL of ethanol was slowly added to precipitate a modified hyaluronic acid, which was washed three times with 50 mL of 80% ethanol to obtain a precursor of a modified hyaluronic acid having a thiol group. In 25 mL of pure water, 0.25 g of the obtained precursor was dissolved, and 180 mg of tris(2-carboxyethyl)phosphine (TCEP) was added thereto, and a reaction was allowed to proceed overnight. To the resulting reaction liquid, 75 mL of ethanol was slowly added to precipitate a modified hyaluronic acid, which was washed three times with 50 mL of 80% ethanol to obtain a modified hyaluronic acid having a thiol group (thiol group-containing modified HA).

### [Example 3]

### <Method for Producing Modified Hyaluronic Acid Having Maleimide Group>

In 25 mL of pure water, 0.474 g of hyaluronic acid (HYALO-OLIGO manufactured by Kewpie Corporation, average molecular weight: 6,000) was dissolved to obtain a hyaluronic acid solution. To the hyaluronic acid solution, 211 mg of 1-hydroxybenzotriazole (HOBT), 240 mg of 1-(3-dimethylaminopropyl)-3-ethylcarbodiimide hydrochloride (EDC·HCl), and 221 mg of N-(2-aminoethyl)maleimide hydrochloride were added, and a reaction was allowed to proceed overnight. To the resulting reaction liquid, 100 mL of ethanol was slowly added to precipitate a modified hyaluronic acid, which was washed three times with 50 mL of 85% ethanol to obtain a modified hyaluronic acid having a maleimide group (maleimide group-containing modified HA).

### [Examples 4 to 11]

### <Method for Producing Modified Hyaluronic Acid Having Thiol Group>

Thiol group-containing modified HAs of Examples 4 to 11 were produced in the same manner as in Example 1 or Example 2 except that the average molecular weight of hyaluronic acid (raw material HA) used as the raw material and the amount of each of HOBT, EDC·HCl, and cystamine dihydrochloride was changed to the condition shown in Table 1.

**[Table 1]**

| Example | Average molecular weight of raw material HA | HOBT (mg) | EDC·HCl (mg) | Cystamine dihydrochloride (mg) |
|---|---|---|---|---|
| 4 | 300,000 | 14 | 15 | 18 |
| 5 | 300,000 | 27 | 30 | 36 |
| 6 | 300,000 | 424 | 480 | 564 |
| 7 | 1,600,000 | 106 | 120 | 141 |
| 8 | 800,000 | 106 | 120 | 141 |
| 9 | 80,000 | 106 | 120 | 141 |
| 10 | 10,000 | 106 | 120 | 141 |
| 11 | 6000 | 106 | 120 | 141 |

The raw material HAs having average molecular weights of 300,000, 1,600,000, 800,000, 80,000, 10,000, and 6,000, respectively, used in production in Examples 4 to 11 were the following hyaluronic acids.
Average molecular weight 300,000: Hyabest (S) LF-P manufactured by Kewpie Corporation
Average molecular weight 1,600,000: Hyaluronsan HA-LQH manufactured by Kewpie Corporation
Average molecular weight 800,000: Hyabest (J) manufactured by Kewpie Corporation
Average molecular weight 80,000: Hyaluronsan HA-LQL manufactured by Kewpie Corporation
Average molecular weight 10,000: Hyaluronsan HA-LF5-A manufactured by Kewpie Corporation
Average molecular weight 6,000: HYALO-OLIGO manufactured by Kewpie Corporation

### [Examples 12 to 15]

### <Method for Producing Modified Hyaluronic Acid Having Maleimide Group>

Maleimide group-containing modified HAs of Examples 12 to 15 were produced in the same manner as in Example 3 except that the average molecular weight of hyaluronic acid (raw material HA) used as the raw material and the amount of each of HOBT, EDC·HCl, and cystamine dihydrochloride was changed to the condition shown in Table 2.

**[Table 2]**

| Example | Average molecular weight of raw material HA | HOBT (mg) | EDC·HCl (mg) | Maleimide hydrochloride (mg) |
|---|---|---|---|---|
| 12 | 6000 | 422 | 480 | 442 |
| 13 | 6000 | 211 | 76 | 70 |
| 14 | 6000 | 26 | 30 | 26 |
| 15 | 10,000 | 211 | 240 | 221 |

The raw material HAs having average molecular weights of 10,000 and 6,000, respectively, used in production in Examples 12 to 15 were the following hyaluronic acids.
Average molecular weight 10,000: Hyaluronsan HA-LF5-A manufactured by Kewpie Corporation
Average molecular weight 6,000: HYALO-OLIGO manufactured by Kewpie Corporation

### <Measurement of Modification Rate>

### (Sample Preparation)

In 0.7 mL of heavy water, 7 mg of a sample and 1 mg of sodium 4,4-dimethyl-4-silapentanesulfonate (DSS) as an internal standard substance were dissolved, and the resultant was transferred to an NMR sample tube, which was capped.

### (Measurement Conditions)

Apparatus: Varian NMR system 400NB (Varian Technologies Japan Limited)
Observation frequency: 400 MHz
Temperature: 30°C
Reference: DSS (0 ppm)
Cumulative number: 64 times

### (Analysis Method)

In ¹H-NMR spectra, a peak derived from CH₃ of an N-acetyl group (2.0 ppm) of hyaluronic acid, a peak of a proton derived from CH₂ of cysteamine (2.7 ppm), and a peak of a proton derived from -CH=CH- of maleimide (6.9 ppm) were integrated. The modification rate was determined from the integrated value using the following formula. Thiol group modification rate (%) = (integrated value of peak of proton derived from CH2 at 2.7 ppm/2)/(integrated value of peak of proton derived from CH3 at 2.0 ppm/3) × 100 Maleimide group modification rate (%) = (integrated value of peak of proton derived from -CH=CH- at 6.9 ppm/2)/(integrated value of peak of proton derived from CH3 at 2.0 ppm/3) × 100

### <Method for Measuring Average Molecular Weight>

The thiol group-containing modified HA or the maleimide group-containing modified HA was dissolved in a mobile phase at a concentration of 0.1%, and the average molecular weight was relatively measured by the following liquid chromatography analysis. A plurality of (purified) hyaluronic acids having known average molecular weights were used as reference substances, a calibration curve was prepared from the retention times thereof, and the average molecular weight of the thiol group-containing modified HA or the maleimide group-containing modified HA was calculated.
Column: TSK Guard Column PWXL + TSK Gel GMPW × 2
Column temperature: 40°C
Measurement wavelength: 210 nm
Flow rate: 0.8 mL/min
Sample injection amount: 20 µL
Analysis time: 40 minutes
Mobile phase: 0.003 mol/L phosphate buffer-0.15 mol/L NaCl (pH 7.0)
Photodiode array: 996 photodiode array manufactured by Nihon Waters K.K.
HPLC system: 2690 separation module manufactured by Nihon Waters K.K.

### [Test Examples]

### <Method for Producing Gel Composition>

A gel composition was produced by a method of mixing any of the thiol group-containing modified HA aqueous solutions of Examples 1, 2 and 4 to 11 with any of the maleimide group-containing modified HA aqueous solutions of Examples 3 and 12 to 15. The thiol group-containing modified HA in an amount shown in Table 3 was dissolved in phosphate buffered saline (PBS) or water to obtain a thiol group-containing modified HA aqueous solution. The maleimide group-containing modified HA in an amount shown in Table 3 was dissolved in PBS or water to obtain a maleimide group-containing modified HA aqueous solution. In Test Example 25, the maleimide group-containing modified HA was used in the form of a powder as it is without being dissolved in PBS or water. In Test Examples 1 to 9, 12 to 23, and 25, when the maleimide group-containing modified HA aqueous solution or the powder of the maleimide group-containing modified HA was added dropwise to the thiol group-containing modified HA aqueous solution, gelling occurred immediately. Gelling in this case proceeded immediately after mixing of the thiol group-containing modified HA aqueous solution and the maleimide group-containing modified HA aqueous solution. The temperature during mixing means both temperatures when the thiol group-containing modified HA aqueous solution and the maleimide group-containing modified HA aqueous solution or the powder of the maleimide group-containing modified HA are mixed. The room temperature shown in Table 3 is 23°C.

**[Table 3]**

| | Thiol group-containing modified HA | | | | | | Maleimide group-containing modified HA | | | | | | Temperature during mixing | Gelling time |
|---|---|---|---|---|---|---|---|---|---|---|---|---|---|---|
| | Example | Modification rate | Average molecular weight | Amount of modified HA | Amount of solvent | Solvent | Example | Modification rate | Average molecular weight | Amount of modified HA | Amount of solvent | Solvent | | |
| Test Example 1 | 1 | 33% | 120,000 | 25 mg | 2.5 mL | PBS | 3 | 16% | 10,000 | 25 mg | 2.5 mL | PBS | Room temperature | Immediately |
| Test Example 2 | | | | 12.5 mg | | | | | | 12.5 mg | | | | |
| Test Example 3 | 2 | 20% | 160,000 | 25 mg | | | | | | 25 mg | | | | |
| Test Example 4 | 4 | 7% | 190,000 | 25 mg | 2.5 mL | PBS | 3 | 18% | 6000 | 25 mg | 2.5 mL | PBS | Room temperature | Immediately |
| Test Example 5 | 5 | 13% | 180,000 | 25 mg | 2.5 mL | PBS | 3 | 18% | 6000 | 25 mg | 2.5 mL | PBS | Room temperature | Immediately |
| Test Example 6 | 2 | 20% | 160,000 | 25 mg | 2.5 mL | PBS | 3 | 16% | 6000 | 25 mg | 2.5 mL | PBS | Room temperature | Immediately |
| Test Example 7 | 1 | 33% | 120,000 | 25 mg | 2.5 mL | PBS | 3 | 16% | 6000 | 25 mg | 2.5 mL | PBS | Room temperature | Immediately |
| Test Example 8 | 6 | 43% | 140,000 | 25 mg | 2.5 mL | PBS | 3 | 18% | 6000 | 25 mg | 2.5 mL | PBS | Room temperature | Immediately |
| Test Example 9 | 1 | 22% | 180,000 | 25 mg | 2.5 mL | PBS | 12 | 24% | 5000 | 25 mg | 2.5 mL | PBS | Room temperature | Immediately |
| Test Example 10 | 1 | 22% | 180,000 | 25 mg | 2.5 mL | PBS | 13 | 9% | 5000 | 25 mg | 2.5 mL | PBS | Room temperature | 5min |
| Test Example 11 | 1 | 22% | 180,000 | 25 mg | 2.5 mL | PBS | 14 | 2% | 6000 | 25 mg | 2.5 mL | PBS | Room temperature | 10 min |
| Test Example 12 | 7 | 23% | 520,000 | 25 mg | 2.5 mL | PBS | 3 | 18% | 6000 | 25 mg | 2.5 mL | PBS | Room temperature | Immediately |
| Test Example 13 | 8 | 25% | 400,000 | 25 mg | 2.5 mL | PBS | 3 | 18% | 6000 | 25 mg | 2.5 mL | PBS | Room temperature | Immediately |
| Test Example 14 | 9 | 24% | 80,000 | 25 mg | 2.5 mL | PBS | 3 | 20% | 5000 | 25 mg | 2.5 mL | PBS | Room temperature | Immediately |
| Test Example 15 | 10 | 14% | 10,000 | 25 mg | 2.5 mL | PBS | 3 | 20% | 5000 | 25 mg | 2.5 mL | PBS | Room temperature | Immediately |
| Test Example 16 | 11 | 23% | 5000 | 25 mg | 2.5 mL | PBS | 3 | 20% | 5000 | 25 mg | 2.5 mL | PBS | Room temperature | Immediately |
| Test Example 17 | 1 | 22% | 180,000 | 25 mg | 2.5 mL | PBS | 15 | 19% | 9000 | 25 mg | 2.5 mL | PBS | Room temperature | Immediately |
| Test Example 18 | 1 | 33% | 120,000 | 12.5 mg | 2.5 mL | PBS | 3 | 16% | 6000 | 12.5 mg | 2.5 mL | PBS | Room temperature | Immediately |
| Test Example 19 | 1 | 22% | 180,000 | 5 mg | 2.5 mL | PBS | 3 | 18% | 6000 | 5 mg | 2.5 mL | PBS | Room temperature | Immediately |
| Test Example 20 | 1 | 22% | 180,000 | 12.5 mg | 1.25 mL | PBS | 3 | 18% | 6000 | 37.5 mg | 3.75 mL | PBS | Room temperature | Immediately |
| Test Example 21 | 1 | 22% | 180,000 | 37.5 mg | 3.75 mL | PBS | 3 | 18% | 6000 | 12.5 mg | 1.25 mL | PBS | Room temperature | Immediately |
| Test Example 22 | 1 | 22% | 180,000 | 25mg | 2.5 mL | PBS | 3 | 18% | 6000 | 25 mg | 2.5 mL | PBS | 37°C | Immediately |
| Test Example 23 | 1 | 22% | 180,000 | 25mg | 2.5 mL | PBS | 3 | 18% | 6000 | 25 mg | 2.5 mL | PBS | 4°C | Immediately |
| Test Example 24 | 1 | 22% | 180,000 | 25mg | 2.5 mL | Water | 3 | 18% | 6000 | 25 mg | 2.5 mL | Water | Room temperature | 30 min |
| Test Example 25 | 1 | 22% | 180,000 | 25mg | 5.0 mL | PBS | 3 | 18% | 6000 | 25 mg | Powder as it is | None | Room temperature | Immediately |

## Claims

1. A material for gel formation, comprising:
at least one component A selected from the group consisting of a modified hyaluronic acid having a thiol group and a salt thereof; and
at least one component B selected from the group consisting of a modified hyaluronic acid having a maleimide group and a salt thereof.

2. The material for gel formation according to claim 1, wherein a thiol group modification rate of the component A is 5% or more and 50% or less.

3. The material for gel formation according to claim 1 or 2, wherein the component A has an average molecular weight of 10,000 or more and 600,000 or less.

4. The material for gel formation according to claim 1 or 2, wherein a maleimide group modification rate of the component B is 1% or more and 30% or less.

5. A material for in situ gel formation, comprising at least one selected from the group consisting of a modified hyaluronic acid having a thiol group and a salt thereof.

6. A material for in situ gel formation, comprising at least one selected from the group consisting of a modified hyaluronic acid having a maleimide group and a salt thereof.

7. A gel composition, comprising:
at least one component A selected from the group consisting of a modified hyaluronic acid having a thiol group and a salt thereof; and
at least one component B selected from the group consisting of a modified hyaluronic acid having a maleimide group and a salt thereof, wherein
the thiol group and the maleimide group are bonded to each other.
